Europäisches Patentamt

European Patent Office

Office européen des brevets

(19)

(11) Publication number: **0 387 739**
**A1**

(12) **EUROPEAN PATENT APPLICATION**

(21) Application number: **90104612.8**

(22) Date of filing: **12.03.90**

(51) Int. Cl.5: **C12P 1/00, C09B 61/00, A61K 47/46, A23L 1/275, //C12P19/60**

(30) Priority: **14.03.89 JP 62967/89**

(43) Date of publication of application:
**19.09.90 Bulletin 90/38**

(84) Designated Contracting States:
**AT BE CH DE DK ES FR GB GR IT LI LU NL SE**

(71) Applicant: **TAKEDA CHEMICAL INDUSTRIES, LTD.**
**3-6, Doshomachi 2-chome Chuo-ku**
**Osaka 541(JP)**

(72) Inventor: **Komiya, Takeya**
**7-43 Minamisakurazuka 1-chome**
**Toyonaka, Osaka 560(JP)**
Inventor: **Yoshida, Shigeru**
**21-16 Jusohonmachi 1-chome, Yodogawa-ku**
**Osaka 532(JP)**
Inventor: **Ozaki, Kazuo, 4-104, 1-4,**
**Koaza-saihoji**
**Aza-enmyoji, Ohyamazaki-cho**
**Otokuni-gun, Kyoto 618(JP)**

(74) Representative: **Lederer, Franz, Dr. et al**
**Lederer, Keller & Riederer, Patentanwälte,**
**Lucile-Grahn-Strasse 22**
**D-8000 München 80(DE)**

(54) **Production method for natural red pigment.**

(57) The present invention provides a production method for a natural red pigment characterized in that the plant of the apple or the like producing the red pigment is cultured to induce a callus which forms a red pigment, the resulting callus being cultured in liquid medium, preferably in the dark, to obtain an uniform cell dispersion, which is then cultured, preferably under blue light irradiation, the red pigment being collected from the resulting culture.
Since the natural red pigment obtained in accordance with the method of the present invention is harmless to humans, it can be used as a colorant in foods, pharmaceuticals and other articles.

## Production Method for Natural Red Pigment

## BACKGROUND OF THE INVENTION

### 1. Field of the Invention

The present invention relates to a production method for a natural red pigment using a callus induced in the tissue of a plant of the genus *Malus* or the like. Since the natural red pigment obtained in accordance with the method of the present invention is harmless to humans, it can be used as a colorant in foods, pharmaceuticals and other articles.

### 2. Description of the Prior Art

In recent years, the use of natural colorants in foods, pharmaceuticals and other articles has expanded, while the use of synthetic colorants, both in amount and in type variety, has declined due to safety concerns. Development of natural color pigments is particularly desired.

Meanwhile, the supply and price of natural colorants are unstable because their supply depends largely on import. To overcome this drawback, attempts have recently been made to produce natural pigments by tissue culture. In comparison with ordinary plant cultivation, tissue culture permits well-controlled production of natural pigments in a shorter time without influence by weather or other conditions.

As such a red pigment, an anthocyanine pigment (cyanidin-3-galactoside) has been extensively studied. The apple plant *Malus pumila* Mill. var. *domestica* C. K. Schn., which contains the anthocyanine pigment in its peel, is particularly drawing attention as a potential source of red pigment; however, this anthocyanine pigment is difficult to utilize as a colorant because it is localized in only several layers of epidermal cells.

Reported studies of tissue culture of the apple plant include the work of R. K. Ibrahim et al. [Lloydia, *34*, 175-182 (1971)] and that of Ota et al. [Journal of the Japanese Society for Horticultural Science, *52*, 117-122 (1983)]; it is known that apple cotyledon- or flesh-derived calluses produce an anthocyanine pigment.

It should be noted, however, that these studies all used a solid medium, not liquid medium, which has been found for more suitable to mass culture by the present inventors. They have also found that a culture using a liquid medium necessitates uniform cell dispersion. Additionally they have found optimum culturing conditions for enhancement of productivity in the production of pigment, but no such conditions are known for cultured apple cells.

Accordingly, no method is known of efficiently producing the red pigment of the apple plant, in large amounts and in a short time, using a liquid medium.

## DESCRIPTION OF PREFERRED EMBODIMENTS

The above-mentioned problems have been solved by the present invention. Accordingly, the present invention provides a production method for a natural red pigment characterized in that the plant of the apple or the like producing the red pigment is cultured to induce a callus which forms a red pigment, the resulting callus being cultured in liquid medium, preferably in the dark, to obtain uniform cell dispersion, which is then cultured, preferably under blue light irradiation, the red pigment being collected from the resulting culture.

In the present invention, a callus induced from the apple plant of the apple or the like producing the red pigment is cultured under light, a red-pigment-producing strain is selected and subjected to liquid suspension culture in the dark to obtain uniformly dispersed cells, which are cultured under blue light to produce the red pigment; the present invention comprises roughly four processes, namely: callus induction, selection, dispersed cell proliferation and pigment production.

The present invention is hereinafter described in the order of process arrangement.

## Callus induction process

A callus can be induced from a tissue of the plant of the apple or the like and cultured by a known method. Examples of plant tissues which can be used include shoots, leaves, fruits and roots; it is preferable to use a piece of a growth portion such as shoot apex, cotyledon or hypocotyl of these tissues. This piece is placed on a liquid medium or solid medium and cultured with lighting until a callus is induced.

It is recommended that the above-mentioned plant be placed on the culture medium in small pieces after sterilization with a disinfectant such as disinfecting alcohol, sodium hypochlorite solution, or filtrate of bleaching powder suspension and washing with sterile water.

Examples of culture media used include ordinary solid media and liquid media for plant tissue culture such as Murashige-Skoog's (hereinafter abbreviated MS) medium, Gamborg's B5 (hereinafter abbreviated B5) medium, Linsmaier-Skoog's medium, White's medium, Heller's medium, Schenk and Hildebrandt's medium, Nitsch and Nitsch's medium and their modifications. It is also possible to use solid media obtained by adding a solidifier (e.g. agar, agarose, Gelrite) to these liquid media.

These media may be formulated with carbon sources, nitrogen sources, inorganic salts, organic substances and other substances as appropriate.

Examples of carbon sources include sugars such as sucrose, glucose, galactose, fructose and maltose, as well as soluble starch. Examples of nitrogen sources include nitrates and ammonium salts. Examples of inorganic salts include those containing elements such as phosphorus, potassium, calcium, magnesium, manganese, copper, zinc, molybdenum, boron, iron, cobalt and nickel. Examples of organic substances include vitamins such as inositol, nicotine, pyridoxine hydrochloride, thiamine hydrochloride, calcium pantothenate, folic acid, paminobenzoic acid, biotin, choline chloride, riboflavin, ascorbic acid, vitamin A, vitamin $D_3$ and vitamin $B_{12}$; organic acids such as sodium pyruvate, citric acid, malic acid and fumaric acid; and naturally occurring substances such as coconut milk, casein hydrolysates and yeast extracts.

The medium may be further supplemented with plant growth regulators such as auxins and cytokinines as well as agar and other substances as appropriate. Examples of auxins which can be used include 2,4-dichlorophenoxyacetic acid, 2,4-dichlorophenylacetic acid, indole-3-acetic acid, indole-3-butyric acid, 1-naphthaleneacetic acid (hereinafter abbreviated NAA), 2-naphthoxyacetic acid, p-chlorophenoxyacetic acid, 2,4,5-trichlorophenoxyacetic acid and 1-naphthaleneacetamide. Examples of cytokinins which can be used include 6-benzyladenine (hereinafter abbreviated BAP), 2-isopentyladenine, 2-isopentenyladenine, kinetin, zeatin, dihydrozeatin, zeatinriboside and diphenylurea. Of these auxins, NAA, for instance, is often used; of these cytokinins, BAP, for instance, is often used.

Auxins are normally added to the culture medium at a ratio of about 0.01 to 20 ppm; cytokinins are normally added to the culture medium at a ratio of about 0.01 to 15 ppm. When cultivation is conducted at about 15 to 35° C in a bright place, callus induction from the tissue piece occurs some 10 to 40 days later.

It is, of course, possible to add inorganic or organic acids, alkalis, buffers and other substances for the purpose of pH adjustment, or appropriate amounts of oils, fats, surfactants and other substances for the purpose of defoaming.

Cultivation can be achieved by stationary culture, shaking culture or aerobic submerged culture. For large scale treatment, what is called shaking culture is desirable. Although cultivating conditions vary depending on the state and composition of the medium, culture method and other aspects, it is normally preferable to cultivate at a temperature of 10 to 45° C and an initial pH of around 7. It is particularly desirable that the mid-cultivation temperature be 20 to 37° C and the initial pH be 5.0 to 8.5. Cultivation time normally ranges from about 1 day to 3 months; preferably 1 to 4 weeks.

## Cell selection process

The formed red-colored callus is cut from the tissue piece and transplanted to the same solid medium or liquid medium as that used for callus induction. Cultivation can be conducted under the same conditions as in the callus induction process. After cultivation, the deep red portion of the callus is further cultured to yield a red pigment strain with an increased pigment concentration. This cycle is repeated at intervals of 5 to 60 days, preferably 14 days, for subculture selection. Usually, a callus which stably forms the red pigment is obtained after 1 to 5 months. This callus is selected for the next process.

## Dispersed cell proliferation process

The obtained callus which stably forms the red pigment is transferred to the above-mentioned liquid medium and subcultured, preferably in the dark, in 2 to 10 cycles at intervals of 5 to 14 days, for cell proliferation. After about 3 weeks, a friable uniformly dispersed cell mass is obtained. This cell mass, after being pulverized into fine cell particles in liquid, is subjected to suspension culture for further cell proliferation. The same liquid medium as that used for callus induction, preferably B5 medium, is used. Cultivation can be conducted under the same conditions as those used for callus induction.

Rapid cell proliferation occurs during this cultivation, which permits mass cultivation.

The obtained cell mass is subcultured at intervals of 5 to 14 days. It is preferable to use the cell mass after sieving. After about 2 to 3 weeks, the cells which proliferated are collected for use as starting cells for the next red pigment production process.

It is preferable that the present process be carried out in the dark, since the ratio of red color cells decreases due to gradual increase in pectin-like substances if it is carried out in a bright place.

Red pigment production process

The starting cells obtained in the previous process are cultured in the same solid medium or liquid medium as that used for callus induction. Liquid media, for example, MS medium, are preferred.

Cultivation time is normally 3 to 14 days, preferably 5 to 10 days. Cultivation temperature is about 10 to 30° C.

Cultivation is conducted with lighting, preferably under irradiation with blue light of a wavelength of 220 to 500 nm. Red to dark red calluses are obtained by this cultivation. These calluses contain a large amount of red pigment. The calluses are collected and, after being squeezed or pulverized as necessary, are extracted with an appropriate solvent; this is followed by solvent removal to yield the red pigment. The collected calluses may be subjected to pigment extraction without drying, or extraction after drying by an ordinary means such as reduced pressure drying. Alcohols such as methanol and ethanol are the desired extraction solvents, preferably containing hydrochloric acid; methanol containing 0.05 to 2.0 w/w% hydrochloric acid is ideal. Temperature is preferably about 0 to 20° C and extraction time about 16 hours to 1 week, in the dark.

The red pigment thus produced may be further purified as desired by known means such as redissolution, concentration (preferably under reduced pressure), chromatography and crystallization.

Production method for beverage or non-beverage food containing red pigment

A beverage or non-beverage food containing red pigment derived from the method of the present invention can easily be produced by formulating the red pigment powder obtained by the method described above in fruit juice, glucose, fructose, liquid sugar or starch syrup.

The beverage or non-beverage food containing a red pigment produced by the method of the present invention may take any form, including juice drinks, ice cream, jelly, jam and drops.

There is no definite specification for the amount of the red pigment used in certain beverage or non-beverage food containing a red pigment produced by the method of the present invention, but it is normally about 0.001 to 20% relative to the entire amount of the finished beverage or non-beverage food.

Production method for a pharmaceutical preparation containing red pigment

A pharmaceutical preparation containing red pigment derived from the method of the present invention can be produced in exactly the same manner as in the production of conventional pharmaceutical preparations, except that the red pigment is used as a colorant.

Examples of pharmaceutical preparations for the present invention include tablets, granules, grains, sugar-coated tablets, film tablets and hard capsules.

There is no definite specification for the amount of the red pigment used in the pharmaceutical preparation containing red pigment produced by the method of the present invention, but it is normally about 0.001 to 20% relative to the entire amount of the finished pharmaceutical preparation.

Examples

The present invention is hereinafter described in more detail by means of the following examples, where % means percent by weight, unless otherwise stated.

## Example 1

[Callus induction process]

An apical bud or axillary bud of the apple plant (variety: Star King) was sterilized by immersion in 70% ethanol for 5 minutes and then in a 1% sodium hypochlorite solution for 10 minutes, after which it was washed with sterile water five times. Subsequently, the shoot apex was excised under stereomicroscopic view and placed on MS-agar medium (2.0 mg/ℓ NAA, 2.5 mg/ℓ BAP, 9g/ℓ agar, pH 5.8). After cultivation at 25°C with lighting (white fluorescence lamp, 2000lux, 16 hours of daylength) for 3 weeks, the red-colored callus formed on the sectional surface of the shoot apex was separated and transplanted to the above-mentioned MS-agar medium.

[Cell selection process]

Thereafter, the deep red portion of the callus was subcultured for two months at intervals of 7 days until a callus was obtained which stably formed the red pigment.

[Dispersed cell proliferation process]

The callus obtained in the previous process was subjected to shaking culture (60 rpm) in the dark using proliferation medium (B5 liquid medium supplemented with 2.0 mg/ℓ NAA, 2.5 mg/ℓ BAP and 3% sucrose). Cells were repeatedly subcultured at intervals of 7 days until a uniform cell dispersion was obtained after 1 month.

[Red pigment production process]

The dispersed cells were collected by filtration on a nylon mesh screen and transplanted to a 9 cm plastic petri dish containing 20 mℓ of pigment production medium (MS medium supplemented with 2.0 mg/ℓ NAA, 2.5 mg/ℓ BAP and 3% sucrose). The amount of starting cells was set at 20 mg/mℓ. Cultivation was conducted by shaking culture (60 rpm) at 25°C under irradiation with a blue fluorescent lamp (model FL-40SB, produced by NEC Corporation) for 8 days.

For control, shaking culture (60 rpm) was conducted at 25°C under respective irradiation with a green fluorescent lamp (model FL-40SG, produced by NEC Corporation), a red fluorescent lamp (model FL-40SPK produced by NEC Corporation) and a plant growing fluorescent lamp (model FL-40SBR-A, produced by NEC Corporation) for 8 days.

To measure the anthocyanine content of the callus obtained by the above-mentioned cultivation, a part of the callus (400 mg) was collected and extracted with 0.1% methanol hydrochloride (4 mℓ) at 4°C in the dark for 48 hours. After impurity removal by filtration, the absorbance (wavelength 530 nm) of the filtrate was determined. The amount of pigment produced (A × B) was obtained by multiplying the absorbance (A) by the wet weight of the callus (B). Table 1 shows the amounts of pigment produced for each fluorescent lamp type.

Table 1

|  | Fluorescent lamp | A: Absorbance per gram fresh callus | B: Fresh callus weight(g) per petri dish | A × B |
|---|---|---|---|---|
| Inventive | Blue lamp | 8.67 | 3.34 | 28.96 |
| Control | Green lamp | 2.95 | 3.39 | 10.00 |
|  | Red lamp | 1.20 | 3.90 | 4.68 |
|  | Plant growing lamp | 3.14 | 4.62 | 14.51 |

As is evident from Table 1, the greatest amount of anthocyanine was produced under irradiation with blue light, the pigment production being more than two times that in other cases.

Also, the callus-containing culture broth obtained by shaking the culture with blue fluorescent lamp irradiation as above was filtered through a 50 μm nylon mesh to collect the callus, which was then freeze-dried. After 24 g of the dry callus thus obtained was defatted with acetone, it was dissolved in 300 mℓ of methanol containing 1% hydrochloric acid. This solution was passed through an ion exchange column (Dowex 50W-X2, produced by Dow Chemical Co.) so that the pigment is adsorbed by the resin. After column washing with sequential addition of water and methanol to remove impurities such as sugar and lipid, the pigment was eluted with 350 mℓ of methanol containing 5% hydrochloric acid. The pigment fraction thus obtained was concentrated under reduced pressure until its volume became 20 mℓ and then passed through a Sephadex LH-20 column (3 cm × 30 cm). After column washing with sequential addition of water and methanol, the pigment was eluted with 170 mℓ of methanol containing 0.4% hydrochloric acid. The eluate was monitored for absorbance at 254 nm. The main fraction thus obtained was concentrated to dryness using a rotary evaporator. The resulting residue was dissolved in 3 mℓ of methanol. To this solution was added 3 mℓ of ethyl ether. A total of 10 cycles of this procedure was repeated to precipitate the pigment to yield 60 mg of a purified pigment powder.

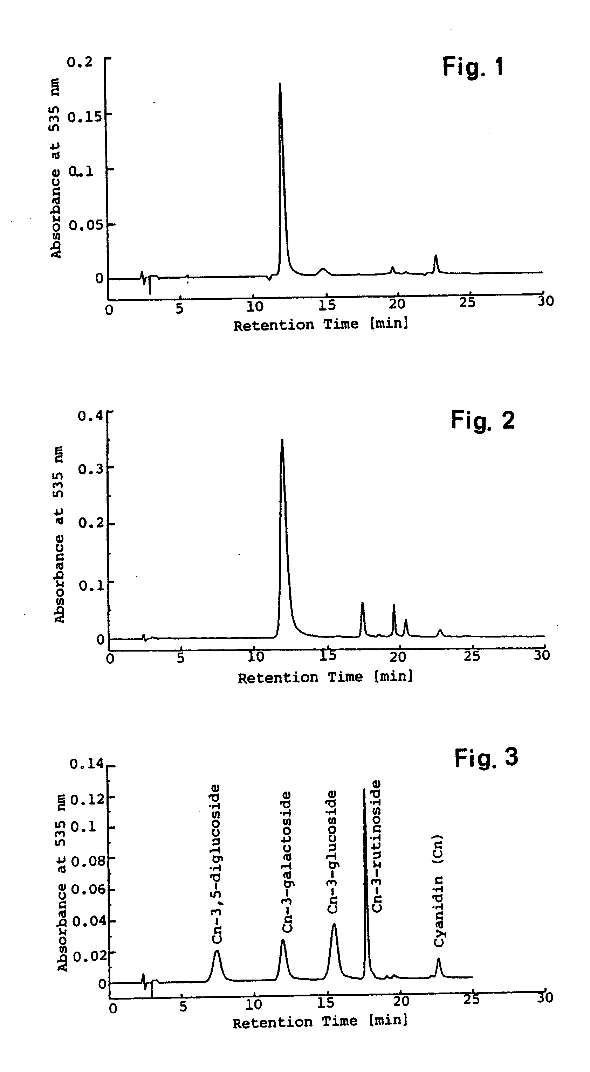

1 mg of the purified pigment powder obtained above was dissolved in 200 μℓ of methanol containing 1% hydrochloric acid. To this solution was added 2 mℓ of 10% formic acid to yield a sample solution, which was subjected to pigment analysis by high performance liquid chromatography (HPLC) as follows: The sample solution was injected into a YMC Pack A312 ODS column (6 mm × 150 mm), through which a mixture of an aqueous solution of 10% formic acid and methanol containing 10% formic acid was transmitted at a flow rate of 1.4 mℓ/min. The ratio of the methanol containing 10% formic acid was 14% (v/v, the same applies below) between 0 and 12 minutes following initiation of analysis, then was changed linearly to 30% from 12 to 16 minutes and was a constant 30% at 16 minutes and thereafter. The eluate was monitored for absorbance at 535 nm. The results are shown in Fig. 1.

For control, 400 mg of a freeze-dried product of apple peels (variety: Star King) was subjected to the same procedure as above to obtain a purified pigment, which was then analyzed by HPLC under the same analytic conditions as above. The results are shown in Fig. 2.

Also used were cyanidin-3,5-diglucoside, cyanidin-3-galactoside, cyanidin-3-glucoside, cyanidin-3-rutinoside and cyanidin as reference substances. The results are shown in Fig. 3.

The highest peak of the apple red pigment produced by the method of the present invention and that of the red pigment purified from apple peels both coincided with the peak of cyanidin-3-galactoside as to peak retention time. The main component of the apple red pigment produced by the method of the present invention was thus identified as the anthocyanin pigment (cyanidin-3-galactoside) contained in apple peels.

Example 2

Cultivation was conducted under the same conditions as in Example 1 except that agar medium (prepared by adding 0.9% agar to the pigment production medium used in the red pigment production process in Example 1) was used. Table 2 shows the amounts of pigment produced after 8 days.

Table 2

| | Fluorescent lamp | A: Absorbance per gram fresh callus | B: Fresh callus weight(g) per petri dish | A × B |
|---|---|---|---|---|
| Inventive | Blue lamp | 3.13 | 0.90 | 2.82 |
| Control | Green lamp | 1.20 | 0.87 | 1.04 |
| | Red lamp | 0.36 | 0.87 | 0.31 |
| | Plant growing lamp | 2.22 | 0.82 | 1.82 |

As is evident from Table 2, a greater amount of anthocyanine was produced under irradiation with blue light than in other cases, though the amounts were about one-tenth in comparison with Example 1 (liquid suspension culture).

Example 3

After immersion in water for 5 hours, 15 g of agar was heated until it dissolved. The resulting agar solution was placed in a kettle, where 380 g of sugar, 50 g of glucose and 425 g of starch syrup were added, and this was followed by vacuum concentration in a vacuum concentrator at 60°C for 15 minutes. Then, the apple red pigment obtained in Example 1 and flavor were added and mixed uniformly with the residue. This mixture was cooled until vapor emission stopped. This liquid was poured into a mold and cooled until it solidified in a solidifying room maintained at 15°C. The obtained solid was removed from the mold and cut into small pieces. Each piece was wrapped with wafer paper to yield jelly having a natural apple color.

Example 4

Apples were washed with water and sliced into 1cm thick pieces. After being immersed in a 2% sodium chloride solution to prevent browning, the fruit pieces were saturated with 100 g of water and boiled down. The boiling-down procedure comprised heating the fruit pieces together with an amount of sugar (400 g) which was half to one-third the total to be added until fruit juice exuded, and the sugar dissolved while stirring in a jacketed kettle and subsequent heating under normal pressure in the presence of the remaining amount of sugar for 20 minutes. After completion of boiling-down, the content was cooled to 80°C in a mechanical cooler, and the apple red pigment obtained in Example 1 was added to yield an apple jam with red color.

Example 5

65 kg of sugar was dissolved in 15 kg of water. To this solution was added 50 kg of starch syrup, and this was followed by boiling-down until the water content became 1 to 2%. Then, 100 g of apple red pigment obtained in Example 1, 500 g of an acidifier and a given amount of flavor were added and mixed with the boiling-down residue on a cooling plate. This mixture was formed using a stamping machine to yield drops with apple red color.

Example 6

7

Red-colored granulation product and granules

1 kg of a powder comprising acetaminophen (40% by weight), lactose (30% by weight) and starch (30% by weight) was charged in a vertical granulator (FM-VG-10, produced by Fuji Sangyo Co., Ltd.), and this was followed by the addition of a mixture of hydroxymethyl cellulose (3% by weight) in a dispersion of 0.1 g of powdered apple pigment obtained in Example 1 in 150 mℓ of water and coloring while granulating to yield a red-colored granulation product.

This granulation product was dried in a vacuum drier (produced by Kusuki Seisakusho Co., Ltd.) and then sized in a power mill (P-3, produced by Showa Kagaku Kikai Co., Ltd.) and sieved to yield granules with a 90% passage rate for a 16 mesh sieve.

Example 7

Red-colored granulation product and tablets

10 kg of a powder composed mainly of ascorbic acid (15% by weight), sodium ascorbate (15% by weight) and sorbitol (60% by weight) was charged in a fluid bed granulator-drier (Glatt WSQ-15, produced by Okawara Seisakusho Co., Ltd.), and this was followed by spraying a dispersion of 20 g of powdered apple pigment obtained in Example 1 in 600 mℓ of water to color the starting powder while granulating.

Subsequently, 100 g of starch was dispersed in 1900 mℓ of water and heated to 80° C. While spraying this glue dispersion, granulation was continued.

The red-colored granulation product was dried and classified, and then made into tablets.

Example 8

Red-colored granulation product

1 kg of crystalline granular sugar was charged in a coating pan (12 inch diameter, produced by Kikusui Seisakusho Co., Ltd.), and this was followed by dust coating with 1 kg of a 1:1 mixture (dusting agent) of ascorbic acid and powdered sugar while spraying on a suspension produced by adding of 0.1 g powdered apple pigment obtained in Example 1 to 500 mℓ of water.

Coating was continued until the grain diameter became about 5 mm, and this was followed by vacuum drying in a vacuum drier (produced by Kusuki Seisakusho Co., Ltd.) to yield a granulation product.

Example 9

Red-colored tablets (sugar-coated tablets)

Tablets (9.5 mm diameter, 250 mg weight, 5,000 tablets) made from a powder composed mainly of lactose (70% by weight) and starch (30% by weight) were coated with sugar in a coating pan (12 inch diameter, produced by Kikusui Seisakusho Co., Ltd.).

The coating solution used was prepared by adding 0.1 g of powdered apple pigment obtained in Example 1 to 1 ℓ of a sugar solution comprising granular sugar, talc, pullulan and water.

Finish coating was conducted with a syrup solution to yield sugar-coated tablets each weighing 450 mg.

Example 10

Red-colored film tablets

Lactose-starch tablets (9.5 mm diameter, 250 mg weight, 40,000 tablets) were film coated with a film solution containing powdered apple pigment obtained in Example 1 using Accelecoater 24 (produced by Manesty Machines).

The film solution used consisted of hydroxypropylmethyl cellulose [TC-5(R); produced by Shin-Etsu Chemical Co. Ltd.], titanium oxide, polyethyleneglycol 6000,0.15 g of powdered apple pigment and 3ℓ of water.

Film tablets each weighing 260 mg were yielded.

Example 11

Red-colored hard capsules

30 parts of gelatin were dissolved in 60 parts of water while heating.

Separately, 1 part of titanium oxide and 1 part of the powdered apple pigment obtained in Example 1 were dispersed in 10 parts of water using the Homomixer (produced by Tokusyu Kika Co.). This dispersion was added to the aqueous solution of gelatin obtained above for coloration.

Capsule pins were placed in this solution, and this was followed by forming and drying to achieve hard capsules.

The obtained capsules had an apple-like bright red color.

Effect of the invention

In accordance with the present invention, a red pigment can be produced on an industrial scale in a short time, without influence by natural conditions such as weather and soil, via tissue culturing of apple callus by a particular method. This pigment can be used as a colorant for foods, pharmaceuticals and other articles, since it is an anthocyanine pigment and thus harmless to humans.

**Claims**

1. A method for artificially producing a natural red pigment characterized by liquid medium cultivation of dispersed cells which produce the red pigment, enabling the subsequent collection thereof.

2. A production method as claimed in Claim 1 characterized by use of dispersed cells produced by subculturing a callus which produces a red pigment on shaking the culture in liquid medium.

3. A production method as claimed in Claim 1 characterized by cultivation of dispersed cells with blue light irradiation.

4. A production method for dispersed cells characterized by subculturing a callus which produces a red pigment on shaking the culture in liquid medium.

5. A production method as claimed in Claim 4 characterized by shaking the culture of a callus in the dark.

6. A beverage or non-beverage food comprising an effective colorant amount of the red pigment obtained by the method in Claim 1.

7. A pharmaceutical comprising an effective colorant amount of the red pigment obtained by the method in Claim 1.

**Fig. 1**

**Fig. 2**

**Fig. 3**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | PATENT ABSTRACTS OF JAPAN, vol. 10, no. 373 (C-391)[2430], 12th December 1986; & JP-A-61 166 396 (NITTO ELECTRIC IND. CO., LTD) 28-07-1986 * The whole abstract * | 1,2,4,5 | C 12 P 1/00<br>C 09 B 61/00<br>A 61 K 47/46<br>A 23 L 1/275 //<br>C 12 P 19/60 |
| Y | IDEM | 3,6,7 | |
| Y | CHEMICAL ABSTRACTS, vol. 100, no. 21, 21st May 1984, page 361, abstract no. 17163q, Columbus, Ohio, US; S. OOTA et al.: "Apple flesh tissue culture and anthocyanin formation in the derived callus tissues", & ENGEI GAKKAI ZASSHI 1983, 52(2), 117-22 * The whole abstract * | 3 | |
| Y | PATENT ABSTRACTS OF JAPAN, vol. 13, no. 171 (C-588)[3519], 24th April 1989; & JP-A-64 2593 (POTSUKA CORP. K.K.) 06-01-1989 * The whole abstract * | 6,7 | TECHNICAL FIELDS SEARCHED (Int. Cl.5) |
| X | PATENT ABSTRACTS OF JAPAN, vol. 12, no. 33 (C-472)[2880], 30th January 1988; & JP-A-62 181 796 (SAN EI CHEM. IND. LTD) 10-08-1987 * The whole abstract * | 1,2,4 | C 12 P<br>C 12 N |
| A | CHEMICAL ABSTRACTS, vol. 75, no. 11, 13th September 1971, page 120, abstract no. 72586s, Columbus, Ohio, US; I. LACKMANN: "Action spectra of anthocyanin synthesis in tissue cultures and seedings of Haplopappus gracilis", & PLANTA 1971, 98(3), 258-69 * The whole abstract * | 3 | |

The present search report has been drawn up for all claims

| Place of search | Date of completion of the search | Examiner |
|---|---|---|
| THE HAGUE | 03-07-1990 | RYCKEBOSCH A.O.A. |